(19) Europäisches Patentamt
European Patent Office
Office européen des brevets

(11) **EP 2 026 717 B1**

(12) **EUROPEAN PATENT SPECIFICATION**

(45) Date of publication and mention
of the grant of the patent:
**25.07.2012 Bulletin 2012/30**

(51) Int Cl.:
*A61F 2/16* (2006.01)     *A61L 27/00* (2006.01)

(21) Application number: **07794802.4**

(22) Date of filing: **11.05.2007**

(86) International application number:
**PCT/US2007/011432**

(87) International publication number:
**WO 2007/142782 (13.12.2007 Gazette 2007/50)**

(54) **A non- or reduced glistenings intraocular lens and method of manufacturing same**

Nicht oder weniger glänzende Intraokularlinse und Herstellungsverfahren dafür

Lentille intraoculaire à scintillement nul ou limité, et procédé de fabrication de celle-ci

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **01.06.2006 US 810303 P**

(43) Date of publication of application:
**25.02.2009 Bulletin 2009/09**

(73) Proprietor: **Advanced Vision Science, Inc.
Goleta, CA 93117 (US)**

(72) Inventors:
• **MENTAK, Khalid
San Ramon, CA 94582 (US)**
• **ALDRED, Margaret
Ventura, CA 93001 (US)**

(74) Representative: **Borchert, Uwe Rudolf et al
Puschmann Borchert Bardehle
Patentanwälte Partnerschaft
Postfach 10 12 31
80086 München (DE)**

(56) References cited:
**EP-A- 0 898 972        WO-A-96/40303
WO-A-2005/047349    US-A- 4 731 079
US-B1- 6 326 448**

## Description

## FIELD

This invention relates to the fields of polymer chemistry, materials science and ophthalmology. More particularly it relates to an intraocular lens and method of preparing same that exhibits reduced or no glistenings when implanted in a patient's eye.

## BACKGROUND

The following is provided as background solely for the benefit of the reader and is not intended, nor is it to be construed, as prior art to the present invention.

The intraocular lens, which can be surgically implanted in the eye of a patient, has experienced a remarkable history of innovation, each predicated on perceived shortcomings of its predecessor. For instance, the first rigid polymethylmethacrylate (PMMA) lens implanted in the posterior chamber between the iris and the lens by Harold Ridley in 1950 resulted in some relatively serious complications such as decentration or delocalization of the lens due to its size and weight and the frequent occurrence of uveitis. In 1953, a few short years after the introduction of the Ridley lens, anterior chamber, the space between the cornea and the iris, implantable lenses were brought to market. These were held in place in the anterior chamber by a closed loop, which, as the name suggests, comprised a string-like piece of non-optic polymeric material attached at both ends to the lens proper, thus forming a loop. The early closed loops, like the lens itself, were rigid. Unfortunately, these lenses, due to their instability in the anterior capsule, were as prone to complications as the Ridley lens with bullous keratopathy, cystoid macular edema and glaucoma being the more common complications observed. To correct the instability problem, anterior chamber lenses were developed that relied on the papillary portion of the iris for anatomical fixation. Some lens designs required suturing to the iris, some clipped on. This, however, was found to lead to luxation of the lens when the pupil dilated unexpectedly. In about 1970, the anterior lens was again restructured, this time using a flexible closed-loop construct. Corneal damage, however, continued to be a problem and corneal transplants due to implant-related damage to the cornea were not uncommon. The next innovation in intraocular lens was the flexible open loop anterior chamber lens. As suggested by the name, "open loop" refers to a non-optic peripheral appendage that is attached to the lens proper at only one point, the other end of the loop being free to move about and conform to the surface of the eye. This resolved for the most part the corneal problems associated with intraocular lenses but other complications such as cystoid macular oedema continued to occur.

In about 1975, the posterior chamber lens was introduced. As mentioned previously, the posterior chamber is the space behind the iris and in front of the eye's natural lens. While the optic portion of this lens, like its predecessors, was made of PMMA, its haptics, that is, the non-optic portion of the lens at the periphery used to hold the lens in place as exemplified by the closed and open loop configurations, were often made of such materials as polyamide or polypropylene. While these lenses offered numerous advantages such as fewer corneal problems, less retinal detachment and less uveitis-glaucoma-hyphaema (UGH) syndrome, they still required relatively large incisions, approaching 6 mm in length, for implantation. Driven by the advent of phacoemulsion technology for the removal of lenses clouded by cataracts through very small incisions in the eye, foldable intraocular lenses were developed. These lenses can be folded to fit through the same incision used to remove the natural lens, i.e., as small as 2.5 - 3.0 mm, and then unfolded to operational size once within the eye. One of the predominant types of foldable intraocular lenses presently in use is the so-called hydrophobic acrylic lens as exemplified by the Alcon Acrysof® lens. These lenses, while relatively new in ophthalmology, seem to be avoiding many, if not most, of the complications of their predecessors.

A problem has arisen with the hydrophobic acrylic lenses, however. The lenses, when implanted in a patient's eye, tend over time to form small, light reflective regions in their structure called "glistenings." While the actual cause of glistenings remains unresolved, one theory is that even though these lenses are nominally hydrophobic, over time some water is able to enter into vacuoles in the polymeric matrix comprising the lens thereby changing the refractive index of the lens at those points, which change appears as reflective spots or "glistenings." While there is still some debate over the effect of glistenings at the clinical level, there remains a concern that in worse case scenarios a loss of visual acuity might occur that may require excision of the lens. Even at lesser levels, glistenings can cause glare and other annoyances to patients who have had a hydrophobic intraocular lens implanted.

WO 2005/047349 discloses copolymers made up of alkoxyalkyl methacrylate and/or alkoxyalkyl acrylate monomers in combination with one or more additional hydrophobic monomers. The copolymers may be used in the manufacture of intraocular lenses, including both the optic and haptic portions of the lenses. WO 2005/ 047349 also discloses methods for making intraocular lenses from the copolymers.

US 6.326.448 discloses a soft intraocular lens material which consists essentially of a polymer obtained by polymerizing polymerizable components containing a hydrophilic monomer, and which has a water absorptivity of from 1.5 to 4.5 wt %.

There is a need for an acrylic foldable intraocular lens that either is not susceptible to glistenings at all or has a substantially reduced tendency to form glistenings.

For this purpose, the intraocular lens according to the invention comprises the features of claim 1. Preferred embodiments of the invention are characterized in the sub-claims.

Thus, the current invention relates to an intraocular lens comprising an optical polymer or polymer blend having an equilibrium water content of from about 1 wt% to about 12 wt% and sufficient isotonic saline to bring the polymer or polymer blend once it has been formed into a lens to its equilibrium water content at a temperature at or above body temperature, that is, at or above approximately 37 °C.

In an aspect of this invention, the intraocular lens is a foldable intraocular lens.

In an aspect of this invention the equilibrium water content of the polymer or polymer blend is from about 2 wt% to about 8 wt%.

In an aspect of this invention, the equilibrium water content of the polymer or polymer blend is from about 3 wt% to about 6 wt%.

In an aspect of this invention the polymer is a copolymer of poly(ethylene glycol) phenyl ether acrylate, styrene, 2-hydroxyethyl methacrylate and ethylene glycol dimethacrylate.

In an aspect of this invention the polymer comprises about 40 wt% poly(ethylene glycol) phenyl ether acrylate, about 26 wt% styrene, about 30 wt% 2-hydroxyethyl methacrylate and about 4 wt% ethylene glycol dimethylacrylate.

In an aspect of this invention, the isotonic saline is selected from the group consisting of balanced salt solution, blood bank saline and phosphate buffered saline.

An aspect of this invention is a method of reducing or eliminating glistenings in an intraocular lens comprising providing an intraocular lens comprising a polymer or polymer blend having an equilibrium water content of from about 1 wt% to about 12 wt% at about 37° C, contacting the intraocular lens with isotonic saline solution at a temperature of about 20 °C to about 90 °C for from about 1 hour to about 36 hours, sterilizing the intraocular lens while maintaining it at its equilibrium water content and implanting the intraocular lens into the eye of a patient in need thereof or storing the intraocular lens in sterile isotonic saline until needed.

An aspect of this invention is the above method wherein the temperature is from about 30 °C to about 80 °C and the time is from about 4 hours to about 30 hours.

An aspect of this invention is the above method wherein the temperature is from about 40 °C to about 70 °C and the time is from about 5 hours to about 24 hours.

An aspect of this invention is the above method wherein the intraocular lens is a foldable intraocular lens.

An aspect of this invention is the above method wherein the intraocular lens is contacted with the isotonic saline solution during manufacture subsequent to solvent extraction and prior to sterilization.

An aspect of this invention is the above method wherein the intraocular lens is contacted with the isotonic saline solution subsequent to sterilization, either during manufacture or after manufacture using sterile isotonic saline solution.

An aspect of this invention is the above method wherein, when the lens is contacted with isotonic saline after manufacture, it has been dried prior to contact with the isotonic saline solution.

An aspect of this invention is the above method wherein the intraocular lens has been commercially packaged prior to contact with the isotonic saline solution.

An aspect of this invention is the above method wherein the equilibrium water content of the polymer or blend of polymers is from about 2 wt% to about 8 wt%.

An aspect of this invention is the above method wherein the equilibrium water content of the polymer or blend of polymers is from about 3 wt% to about 6 wt%.

An aspect of this invention is the above method wherein the polymer is a copolymer comprising poly(ethylene glycol) phenyl ether acrylate, styrene, 2-hydroxyethyl methacrylate and ethylene glycol dimethacrylate.

An aspect of this invention is the above method wherein the polymer comprises about 40 wt% poly(ethylene glycol) phenyl ether acrylate, about 26 wt% styrene, about 30 wt% 2-hydroxyethyl methacrylate and about 4 wt% ethylene glycol dimethylacrylate.

An aspect of this invention is the above method wherein the isotonic saline is selected from the group consisting of balanced salt solution, blood bank saline and phosphate buffered saline.

## DETAILED DESCRIPTION

As used herein, an "intraocular lens" refers to a polymeric phakic or aphakic (also referred to in the art as pseudophakic), vision-correcting device that may be implanted into a patient's eye. Phakic lenses are used to correct refractive errors such as myopia (near-sightedness), hyperopia (farsightedness) and astigmatism (blurred vision due to poor light focusing on the retina due to an irregularly shaped cornea or, in some instances, an irregularly shaped natural lens). The natural lens remains in place when a phakic lens is implanted while the lens is removed prior to implantation of pseudophakic lens. An aphakic or pseudophakic lens is inserted in the eye subsequent to removal of the natural lens due to disease, most often a cataract; that is, clouding of the natural lens. Either type of lens may be implanted in the anterior chamber in front of the iris or in the posterior chamber behind the iris and in front of the natural lens or in the region where the natural lens was before removal. While intraocular lenses may be "hard," that is relatively inflexible, or "soft," i.e., relatively flexible but not foldable, for the purpose of this invention the presently preferred lens is a foldable acrylic polymer lens. A foldable lens is one that is sufficiently flexible that it can be folded into a smaller configuration to permit its implantation into the eye through a much smaller incision that is necessary for hard or soft lenses. That is, while hard and soft lenses may require a 6 mm or larger incision, a foldable lens usually requires only a 3 mm or even smaller incision.

As used herein, the terms "approximately," "essentially," "substantially," "about," "slightly" or any other term of approximation, unless otherwise expressly stated, mean ± 5% from the figure set forth.

As used herein, to "contact" a lens with isotonic saline solution refers preferably to submersing the lens in the solution although it is possible to achieve the same result by merely floating the lens atop the isotonic saline solution. As used herein, a "patient" refers to any sighted species suffering from a disorder related to visual acuity. In particular, a patient is a mammal, most particularly a human being. As used herein, a patient is "in need of" an intraocular lens when the patient's natural lens either passes light only partially or not at all as the result of opacification of the lens, or passes light but does not properly focus it on the retina. Such may occur as the result of natural conditions, i.e., aging, or it may occur as a symptom of another disease such as, without limitation, diabetes.

As used herein, a "polymer" refers to a homopolymer prepared by the polymerization of a single monomer or to a copolymer prepared by the polymerization of two or more different monomers. Copolymers may be random, alternating, ordered block, random block or graft copolymers. To be useful in the method of this invention, however, the polymer or copolymer must have an equilibrium water content at approximately body temperature, i.e., about 37 °C, from about 1 wt% to about 12 wt%, preferably from about 2 wt% to about 8 wt% and presently most preferably from about 3 wt% to about 6 wt%. Such polymers are generally referred to by those of ordinary skill in the intraocular lens art as "hydrophobic polymers," even though they are capable of absorbing and retaining significant amounts of water.

As used herein, "optical polymer" refers to a polymer that is suitable for implantation into a patient's eye and that is capable of addressing ophthalmic conditions of the lens of the eye such as, without limitation, myopia, hyperopia, astigmatism and cataracts. In general such a polymer will be biocompatible, i.e., will not cause any inflammatory, immunogenic, or toxic condition when implanted will form a clear, transparent, colorless (unless intentionally colored for a particular application) film-like membrane and will have a refractive index greater than about 1.4, preferably greater than about 1.5 and presently most preferably greater than about 1.55.

An example of a presently preferred polymer for use in the intraocular lens and method of this invention is a copolymer of poly(ethylene glycol) phenyl ether acrylate, styrene, 2-hydroxyethyl methacrylate and ethylene glycol dimethacrylate (as a cross-linker). In an embodiment of this invention, the monomers are present in the finished polymer at approximately 40 wt%, 26 wt%, 30 wt% and 4 wt%, respectively. This polymer has an equilibrium water content at about 37 °C (body temperature) of approximately 4%.

As used herein "equilibrium water content" refers to the quantity of isotonic saline solution that a polymer, copolymer or blend of polymers and/or copolymers can absorb at a given temperature, stated as a weight percent (wt%) calculated using the formula EWC (%) = 100 x $(M_h - M_d)/M_d$, wherein $M_d$ is the weight of the dry polymer and $M_h$ is the weight of the hydrated polymer. For the purposes of this invention, the equilibrium water content is the amount of water that a polymer can contain at about body temperature, that is, about 37 °C. To achieve the desired equilibrium water content for a polymer of this invention, the polymer is placed in contact with isotonic saline at about 20 °C to about 90 °C for from about 1 to about 36 hours, preferably at about 30 °C to about 80 °C for from about 4 hours to about 30 hours and presently most preferably at about 40 °C to about 70 °C for from about 5 to about 24 hours.

As used herein, "isotonic saline" refers to a salt, normally sodium chloride, dissolved in water, the amount of salt being substantially the same as that in bodily fluids. For use in the eye, this is approximately 0.8-0.9 % w/v (weight per unit volume) of sodium chloride in water. In the metric system, w/v is the same as w/w since a unit volume of water, that is one cubic centimeter, weight one gram. The isotonic saline may be buffered to match intraocular pH by the addition of boric acid and sodium borate or sodium phosphate and potassium phosphate (phosphate-buffered saline, PBS). Presently preferred isotonic saline solutions for use in the intraocular lens and method of this invention are phosphate-buffered saline, such as, without limitation, Dulbecco's buffered phosphate solution; balanced salt solutions such as, again without limitation, Hank's balanced salt solution and Earle's balanced salt solution; and blood bank saline, an approximately 0.85 to 0.9 % sodium chloride solution buffered to blood pH (7.0 - 7.2). Numerous other physiological (i.e., isotonic) saline preparations containing a variety of additional substances are known in the art; any of them that are known or shown to be usable in the eye may be used as the isotonic solution of this invention and all such physiological saline solutions are within the scope of this invention.

An intraocular lens of this invention may be produced as a step in the manufacturing process used to create the lens. For example, without limitation, a manufacturing process may include the steps of polymer synthesis, polymer sheet casting, button cutting, optic lathe cutting, optic milling, haptic attachment, polishing, solvent extraction, sterilization and packaging. The hydration of the lens to equilibrium water content step is currently most preferably, though not necessarily, performed between solvent extraction and sterilization. Hydration to equilibrium water content is accomplished by placing the lens in isotonic saline and heating to from about 20 °C to about 90 °C for from about 1 to about 36 hours, preferably at about 30 °C to about 80 °C for from about 4 hours to about 30 hours and presently most preferably at about 40 °C to about 70 °C for from about 5 to about 24 hours.

It is possible, and it is an embodiment of this invention, to hydrate the lens after sterilization just prior to packaging by using a sterile isotonic saline solution and the above conditions.

It is also possible, and is likewise an embodiment of this invention, to hydrate an intraocular lens of this invention just prior to use. That is, the intraocular lens, current commercial versions of which are normally packaged in the dry state, is removed from its sterile packaging under sterile conditions, placed in sterile isotonic saline and subjected to the above conditions prior to insertion into a patient's eye. If the lens is packaged wet, that is, is already in a sterile isotonic solution, the entire container may be heated to the requisite temperature for the indicated period of time to achieve equilibrium water content prior to implantation in a patient's eye.

As used herein a "commercially packaged" intraocular lens refers to a lens that has been dried and placed in a sterile package for storage until needed. The dry sterile package may be any presently known in the art or as such may become known in the future.

## Claims

1. An intraocular lens, comprising:

   an optical polymer or polymer blend having an equilibrium water content of from about 1 wt% to about 12 wt%; and
   isotonic saline to bring the polymer or polymer blend once it has been formed into a lens to its equilibrium water content at a temperature at or above approximately 37°C,
   where the quantity of the isotonic saline solution to bring the polymer or polymer blend once it has been formed into a lens to its equilibrium water content (EWC) is given by the formula:

   $$EWC\ (\%) = 100 \times (M_h - M_d)\ /\ M_d$$

   where $M_d$ is the weight of the dry polymer and $M_h$ is the weight of the hydrated polymer.

2. The intraocular lens of claim 1, wherein the lens is a foldable intraocular lens.

3. The intraocular lens of claim 1, wherein the equilibrium water content of the polymer or polymer blend is from about 2 wt% to about 8 wt%, preferably from about 3 wt% to about 6 wt%.

4. The intraocular lens of claim 1, wherein the optical polymer or polymer blend is a copolymer of poly(ethylene glycol) phenyl ether acrylate, styrene, 2-hydroxyethyl methacrylate and ethylene glycol dimethacrylate.

5. The intraocular lens of claim 4, wherein the copolymer comprises about 40 wt% poly(ethylene glycol) phenyl ether acrylate, about 26 wt% styrene, about 30 wt% 2-hydroxyethyl methacrylate and about 4 wt% ethylene glycol dimethylacrylate.

6. The intraocular lens of claim 1, wherein the isotonic saline is selected from the group consisting of balanced salt solution, blood bank saline and phosphate buffered saline.

7. A method of reducing or eliminating glistenings in an intraocular lens, comprising: providing an intraocular lens comprising a polymer or polymer blend having an equilibrium water content of from about 1 wt% to about 12 wt% at about 37°C; contacting the intraocular lens with isotonic saline solution at a temperature of about 20°C to about 90°C for from about 1 hour to about 36 hours, , where the isotonic saline preferably is selected from the group consisting of balanced salt solution, blood bank saline and phosphate buffered saline; sterilizing the intraocular lens while maintaining it at its equilibrium water content; and storing the intraocular lens in sterile isotonic saline until needed for implantation into the eye of a patient in need thereof.

8. The method of claim 7, wherein the temperature of the isotonic saline solution is from about 30°C to about 80°C and the contact time is from about 4 hours to about 30 hours, preferably from about 40°C to about 70°C and the contact time is from about 5 hours to about 24 hours.

9. The method of claim 7, wherein the intraocular lens is contacted with the isotonic saline solution during manufacture subsequent to solvent extraction and prior to sterilization.

10. The method of claim 7, wherein the intraocular lens is contacted with the isotonic saline solution subsequent to sterilization, either during manufacture or after manufacture using sterile isotonic saline solution.

11. The method of claim 10, wherein when the lens is contacted with isotonic saline after manufacture, it has been dried prior to contact with the isotonic saline solution.

12. The method of claim 8, wherein the intraocular lens has been commercially packaged prior to contact with the isotonic saline solution.

**13.** The method of claim 8, wherein the equilibrium water content of the polymer or blend of polymers is from about 2 wt% to about 8 wt% at about 37°C, preferably from about 3 wt% to about 6 wt% at about 37°C.

**14.** The method of claim 8, wherein the polymer or polymer blend comprises a copolymer comprising poly (ethylene glycol) phenyl ether acrylate, styrene, 2-hydroxyethyl methacrylate and ethylene glycol dimethacrylate.

**15.** The method of claim 14, wherein the copolymer comprises about 40 wt% poly(ethylene glycol) phenyl ether acrylate, about 26 wt% styrene, about 30 wt% 2-hydroxyethyl methacrylate and about 4 wt% ethylene glycol dimethylacrylate.

**Patentansprüche**

**1.** Intraokularlinse umfassend:

ein optisches Polymer oder eine Polymermischung mit einem Gleichgewichts-Wassergehalt von etwa 1 Gew.-% bis etwa 12 Gew.-%; und

eine isotonische Salzlösung, um das Polymer oder die Polymermischung, nachdem sie in eine Linse geformt ist, auf den Gleichgewichts-Wassergehalt bei einer Temperatur bei oder oberhalb von etwa 37 °C zu bringen,

worin die Menge an isotonischer Salzlösung, um das Polymer oder die Polymermischung, nachdem sie in die Form einer Linse gebracht ist, auf ihren Gleichgewichts-Wassergehalt (EWC) zu bringen, gegeben ist durch die folgende Formel:

$$EWC\ (\%) = 100 \times (M_h - M_d)\ /\ M_d$$

wobei $M_d$ das Gewicht des trockenen Polymers und $M_h$ das Gewicht des hydrierten Polymers ist.

**2.** Intraokularlinse nach Anspruch 1, worin die Linse eine faltbare Intraokularlinse ist.

**3.** Intraokularlinse nach Anspruch 1, wobei der Gleichgewichts-Wassergehalt des Polymers oder der Polymermischung von etwa 2 Gew.-% bis etwa 8 Gew.-% liegt, vorzugsweise von etwa 3 Gew.-% bis etwa 6 Gew.-%.

**4.** Intraokularlinse nach Anspruch 1, wobei das optische Polymer oder die Polymermischung ein Copolymer ist von Poly(ethylenglycol-)phenyletheracrylat, Styrol, 2-Hydroxyethylmethacrylat und Ethylenglycoldimethacrylat.

**5.** Intraokularlinse nach Anspruch 4, worin das Copolymer etwa 40 Gew.-% Poly(ethylenglycol-) phenyletheracrylat, etwa 26 Gew.-% Styrol, etwa 30 Gew.-% 2-Hydroxyethylmethacrylat und etwa 4 Gew.-% Ethylenglycoldimethylacrylat umfasst.

**6.** Intraokularlinse nach Anspruch 1, worin die isotonische Salzlösung aus der Gruppe bestehend aus einer Gleichgewichts-Salzlösung, einer Blutbank-Salzlösung und einer mit Phosphat gepufferten Salzlösung ausgewählt ist.

**7.** Verfahren zur Verminderung oder Eliminierung von Glänzen in einer Intraokularlinse umfassend: Bereitstellen einer Intraokularlinse umfassend ein Polymer oder eine Polymermischung mit einem Gleichgewichts-Wassergehalt von etwa 1 Gew.-% bis etwa 12 Gew.-% bei etwa 37 °C, Kontaktieren der Intraokularlinse mit einer isotonischen Salzlösung bei einer Temperatur von etwa 20 °C bis etwa 90 °C während etwa 1 Stunde bis etwa 36 Stunden, wobei die isotonische Salzlösung vorzugsweise ausgewählt ist aus der Gruppe bestehend aus einer Gleichgewichts-Salzlösung, einer Blutbank-Salzlösung und einer Phosphat gepufferten Salzlösung, Sterilisieren der Intraokularlinse während sie bei ihrem Gleichgewichts-Wassergehalt gehalten wird, und Speichern der Intraokularlinse in einer sterilen, isotonischen Salzlösung bis sie zur Implantation in das Auge eines Patienten, der die benötigt, gebraucht wird.

**8.** Verfahren nach Anspruch 7, worin die Temperatur der isotonischen Salzlösung von etwa 30 °C bis etwa 80 °C, die Kontaktzeit von etwa 4 Stunden bis etwa 30 Stunden, vorzugsweise von etwa 40 °C bis etwa 70 °C und die Kontaktzeit von etwa 5 Stunden bis etwa 24 Stunden betragen.

**9.** Verfahren nach Anspruch 7, worin die Intraokularlinse mit der isotonischen Salzlösung während der Herstellung nachfolgend auf die Lösungsmittelextraktion und vor der Sterilisation in Kontakt gebracht wird.

**10.** Verfahren nach Anspruch 7, worin die Intraokularlinse mit der isotonischen Salzlösung nachfolgend auf die Sterilisation entweder während der Herstellung oder nach der Herstellung unter Verwendung der sterilen isotonischen Salzlösung in Kontakt gebracht wird.

**11.** Verfahren nach Anspruch 10, wobei, wenn die Linse mit der isotonischen Salzlösung nach der Herstellung in Kontakt gebracht wird, sie vor dem Kontakt mit der isotonischen Salzlösung getrocknet worden ist.

glycoldimethacrylat.

**12.** Verfahren nach Anspruch 8, wobei die Intraokularlinse kommerziell verpackt worden ist vor dem Kontakt mit der isotonischen Salzlösung.

**13.** Verfahren nach Anspruch 8, worin der Gleichgewichts-Wassergehalt des Polymers oder der Polymermischung von etwa 2 Gew.-% bis etwa 8 Gew.-% bei 37 °C, vorzugsweise von etwa 3 Gew.-% zu etwa 6 Gew.-% bei etwa 37 °C beträgt.

**14.** Verfahren nach Anspruch 8, worin das Polymer oder die Polymermischung ein Copolymer umfasst, welches Poly(ethylenglycol-)phenyletheracrylat, Styrol, 2-Hydroxethylmethylacrylat und Ethylenglycoldimethacrylat umfasst.

**15.** Verfahren nach Anspruch 14, worin das Copolymer etwa 40 Gew.-% Poly(ethylenglycol-) phenyletheracrylat, etwa 26 Gew.-% Styrol, etwa 30 Gew.-% 2-Hydroxyethylmethacrylat und etwa 4 Gew.-% Ethylenglycoldimethylacrylat umfasst.


**Revendications**

**1.** Lentille intraoculaire, comprenant:

- un polymère optique ou le mélange de polymères ayant une teneur en eau d'équilibre entre environ 1% en poids et environ 12% en poids; et
- la saline isotonique pour amener le polymère ou le mélange de polymères une fois qu'il a été formé dans une lentille à sa teneur en eau d'équilibre à une température à ou au-dessus d'environ 37 °C,
où la quantité de la solution de saline isotonique pour amener le polymère ou le mélange de polymères une fois qu'il a été formé dans une lentille à sa teneur en eau d'équilibre (EWC) est donnée par la formule:

$$EWC\ (\%) = 100 \times (M_h - M_d)/M_d$$

où $M_d$ est le poids du polymère sec et $M_h$ est le poids du polymère hydraté.

**2.** Lentille intraoculaire selon la revendication 1, où la lentille est une lentille intraoculaire pliable.

**3.** Lentille intraoculaire selon la revendication 1, où la teneur en eau d'équilibre du polymère ou du mélange de polymères est entre environ 2% en poids et environ 8% en poids, préférablement entre environ 3% en poids et environ 6% en poids.

**4.** Lentille intraoculaire selon la revendication 1, où le polymère optique ou le mélange de polymères est un copolymère de poly(éthylène glycol) phényle éther acrylate, styrène, 2-hydroxyéthyl méthacrylate et diméthacrylate d'éthylène glycol.

**5.** Lentille intraoculaire selon la revendication 4, où le copolymère comprend environ 40% en poids poly(éthylène glycol) phényle éther acrylate, environ 26% en poids styrène, environ 30% en poids 2-hydroxyéthyl méthacrylate et environ 4% en poids diméthacrylate d'éthylène glycol.

**6.** Lentille intraoculaire selon la revendication 1, où la saline isotonique est sélectée du groupe formé d'une solution saline équilibrée, solution saline de banque de sang et solution saline tamponnée de phosphate.

**7.** Procédé de réduction ou d'élimination de scintillements dans une lentille intraoculaire, comprenant: pourvoir une lentille intraoculaire comprenant un polymère ou le mélange de polymères ayant une teneur en eau d'équilibre entre environ 1% en poids et environ 12% en poids à environ 37 °C; mettre en contact la lentille intraoculaire avec la solution saline isotonique à une température d'environ 20 °C à environ 90 °C pour entre environ 1 heure et environ 36 heures, où la saline isotonique est préférablement sélectée du groupe formé d'une solution saline équilibrée, solution saline de banque de sang et solution saline tamponnée de phosphate; stériliser la lentille intraoculaire pendant qu'en la maintenant à sa teneur en eau d'équilibre; et stocker la lentille intraoculaire dans la saline isotonique stérile jusqu'à son utilisation pour implantation dans l'oeil d'un patient la nécessitant.

**8.** Procédé selon la revendication 7, où la température de la solution saline isotonique est entre environ 30 °C et environ 80 °C et le temps de contact est entre environ 4 heures et environ 30 heures, préférablement entre environ 40 °C et environ 70 °C et le temps de contact est entre environ 5 heures et environ 24 heures.

**9.** Procédé selon la revendication 7, où la lentille intraoculaire est en contact avec la solution saline isotonique pendant la fabrication après l'extraction du solvant et avant de stérilisation.

**10.** Procédé selon la revendication 7, où la lentille intraoculaire est en contact avec la solution saline isotonique après la stérilisation, soit pendant la fabrication soit après la fabrication en utilisant la solution saline isotonique stérile.

**11.** Procédé selon la revendication 10, où quand la lentille est en contact avec la solution saline isotonique après la fabrication, elle a été séchée avant le contact avec la solution saline isotonique.

**12.** Procédé selon la revendication 8, où la lentille intraoculaire a été commercialement emballée avant le contact avec la solution saline isotonique.

**13.** Procédé selon la revendication 8, où la teneur en eau d'équilibre du polymère ou mélange de polymères est entre environ 2% en poids et environ 8% en poids à environ 37 °C, préférablement entre environ 3% en poids et environ 6% en poids à environ 37 °C.

**14.** Procédé selon la revendication 8, où le polymère ou le mélange de polymères est un copolymère comprenant poly(éthylène glycol) phényle éther acrylate, styrène, 2-hydroxyéthyl méthacrylate et diméthacrylate d'éthylène glycol.

**15.** Procédé selon la revendication 14, où le copolymère comprend environ 40% en poids poly(éthylène glycol) phényle éther acrylate, environ 26% en poids styrène, environ 30% en poids 2-hydroxyéthyl méthacrylate et environ 4% en poids diméthacrylate d'éthylène glycol.

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005047349 A

- US 6326448 B